(19) Europäisches Patentamt
European Patent Office
Office européen des brevets

(11) EP 0 848 707 B1

(12) **FASCICULE DE BREVET EUROPEEN**

(45) Date de publication et mention de la délivrance du brevet:
**04.08.1999 Bulletin 1999/31**

(21) Numéro de dépôt: **96930216.5**

(22) Date de dépôt: **05.09.1996**

(51) Int Cl.⁶: **C07D 211/12**, A61K 31/445, C07D 401/06

(86) Numéro de dépôt international:
**PCT/FR96/01362**

(87) Numéro de publication internationale:
**WO 97/09309 (13.03.1997 Gazette 1997/12)**

# (54) DERIVES DE 4-(CYCLOALKYL)PIPERIDINES ET DE 4-(CYCLOALKYLALKYL)PI PERIDINES, LEUR PREPARATION ET LEUR APPLICATION EN THERAPEUTIQUE

4-(CYCLOALKYL)PIPERIDINE UND 4-(CYCLOALKYLALKYL)-PIPERIDINE, IHRE HERSTELLUNG UND THERAPEUTISCHE VERWENDUNG

4-(CYCLOALKYL)PIPERIDINES AND 4-(CYCLOALKYLALKYL)-PIPERIDINES DERIVATIVES, PREPARATION THEREOF AND THERAPEUTICAL APPLICATIONS THEREOF

(84) Etats contractants désignés:
**AT BE CH DE DK ES FI FR GB GR IE IT LI LU NL PT SE**
Etats d'extension désignés:
**AL LT LV SI**

(30) Priorité: **08.09.1995 FR 9510512**
**08.09.1995 FR 9510513**

(43) Date de publication de la demande:
**24.06.1998 Bulletin 1998/26**

(73) Titulaire: **SYNTHELABO**
**92350 Le Plessis Robinson (FR)**

(72) Inventeurs:
- **LARDENOIS, Patrick**
  **F-92340 Bourg-la-Reine (FR)**
- **FROST, Jonathan**
  **F-91320 Wissous (FR)**
- **PASAU, Patrick**
  **B-1450 Cortil-Noirmont (BE)**
- **GEORGE, Pascal**
  **F-78730 Saint-Arnoult-en-Yvelines (FR)**
- **RENONES, Maria, Carmen**
  **F-95880 Enghien-les-Bains (FR)**
- **BARTSCH, Régine**
  **F-92260 Fontenay-aux-Roses (FR)**
- **LI, Wai-Tak**
  **F-67200 Strasbourg-Elsau (FR)**
- **MAGAT, Pascale**
  **F-91380 Chilly-Mazarin (FR)**
- **DUPONT, Régis**
  **F-37100 Tours (FR)**

(74) Mandataire: **Ludwig, Jacques et al**
**Sanofi-Synthélabo**
**Service Brevets**
**174, avenue de France**
**75013 Paris (FR)**

(56) Documents cités:
**EP-A- 0 351 282**
**FR-A- 2 684 379**
**US-A- 4 028 366**

- **ARZNEIMITTELFORSCHUNG, vol. 21, no. 12, 1971, pages 1992-1998, XP000567831**
- **J. MED. CHEM. (1983), 26(1), 42-50 CODEN: JMCMAR;ISSN: 0022-2623, 1983, XP000608931 HUEGI, BRUNO S. ET AL: "Synthesis and pharmacological studies of 4,4-disubstituted piperidines: a new class of compounds with potent analgesic properties"**
- **CHEMICAL ABSTRACTS, vol. 123, no. 7, 14 Août 1995 Columbus, Ohio, US; abstract no. 83361, MISHINA, TADASHI ET AL: "Preparation of imidazolylbenzene compounds and use thereof as medicines" XP002017541 & WO 94 18172 A (YOSHITOMI PHARMACEUTICAL INDUSTRIES, LTD., JAPAN)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

Les composés de l'invention répondent à la formule générale (I)

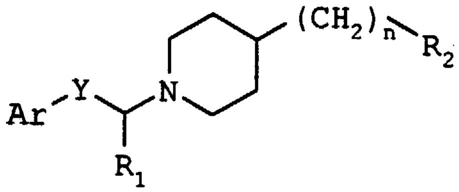

(I)

dans laquelle

Ar représente

a) un groupe phényle substitué par un atome d'halogène ou bien
b) un groupe de formule générale (I')

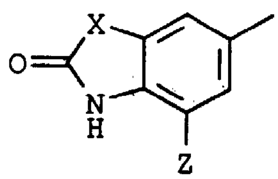

(I')

dans laquelle X représente un groupe de formule $-CH_2-$, $-(CH_2)_2-$ ou $-(CH_2)_3-$ et Z représente un atome d'hydrogène ou d'halogène ou un groupe méthyle,

Y représente un groupe de formule $-CH_2-$, -CO- ou -CHOH-,
$R_1$ représente un atome d'hydrogène ou un groupe méthyle,
$R_2$ représente un groupe cycloalkyle en $C_3$-$C_7$, et
n représente le nombre 0, 1, 2 ou 3.

Les composés de l'invention peuvent exister à l'état de bases libres ou de sels d'addition à des acides.

Par ailleurs, lorsque Y représente un groupe -CHOH-, l'atome de carbone de ce groupe est asymétrique ; de même, lorsque $R_1$ représente un groupe méthyle, l'atome de carbone qui le porte est, lui aussi, asymétrique. Un composé selon l'invention peut donc, selon le cas, se présenter sous la forme d'un isomère optique pur ou d'un mélange de tels isomères.

Conformément à l'invention, on peut préparer les composés de formule générale (I) par des procédés illustrés par les schémas qui suivent.

Selon le schéma 1, on fait d'abord réagir une cétone halogénée de formule générale (II), dans laquelle Ar est tel que défini ci-dessus et Hal représente un atome d'halogène, avec une pipéridine substituée de formule générale (III), dans laquelle n et $R_2$ sont tels que définis ci-dessus, pour obtenir la cétone de formule générale (Ia), laquelle correspond à la formule générale (I) lorsque Y représente un groupe -CO-.
Si on le désire on peut ensuite réduire cette cétone, par exemple au moyen d'un borohydrure de métal alcalin, pour obtenir l'alcool de formule générale (Ib), laquelle correspond à la formule générale (I) lorsque Y représente un groupe -CHOH-.
Les conditions des réactions de ces deux étapes sont bien connues de l'homme du métier.

Pour obtenir un composé de formule générale (I) dans laquelle Y représente un groupe de formule $-CH_2-$ et Ar représente un groupe phényle substitué par un atome d'halogène on peut ensuite réduire l'alcool de formule générale (Ib), par exemple par réaction avec le chlorure de thionyle, suivi du traitement de l'intermédiaire chloré par l'hydrure de lithium et d'aluminium.

Pour obtenir un composé de formule générale (I) dans laquelle Y représente un groupe de formule $-CH_2-$ et Ar représente un groupe de formule générale (I'), et selon le schéma 2, on réduit d'abord une cétone halogénée de formule générale (II), dans laquelle Ar représente un groupe de formule générale (I') et Hal représente un atome d'halogène, au moyen de triéthylsilane et d'acide trifluoroacétique, selon une méthode décrite dans la demande de

brevet EP-0281309, pour obtenir le dérivé halogéné de formule générale (IV), puis on fait réagir ce dernier avec une pipéridine substituée de formule générale (III), dans laquelle n et $R_2$ sont tels que

Schéma 1

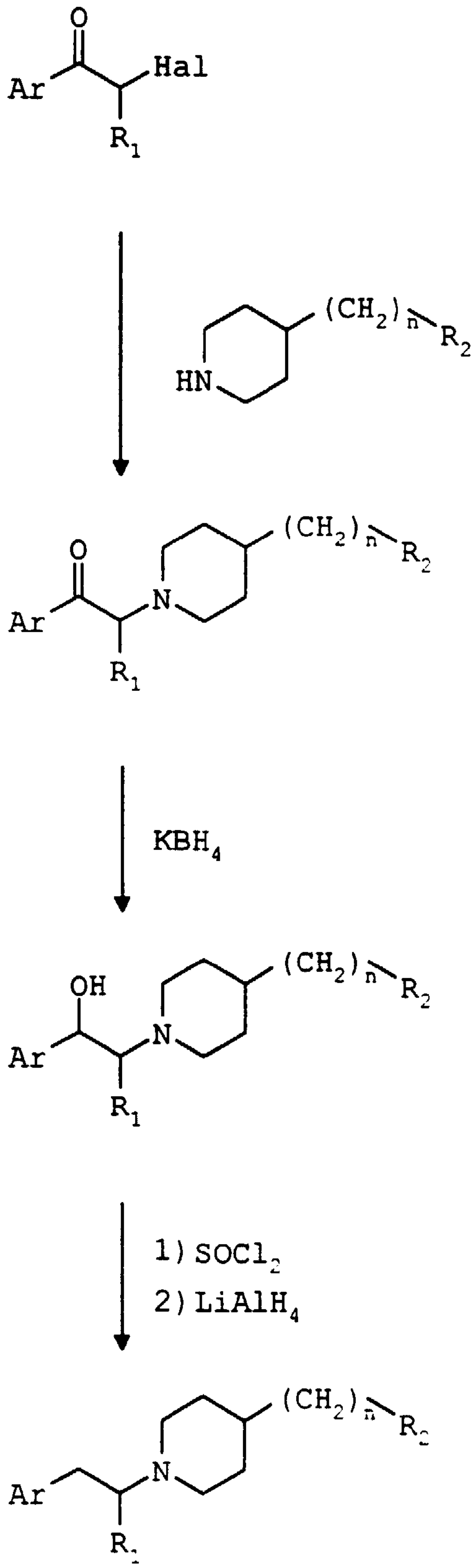


(II)

(III)

(Ia)

(Ib)

(Ic)

définis ci-dessus, dans des conditions classiques, par exemple dans un solvant tel que le *N,N*-diméthylformamide et en présence d'une base telle que le carbonate de sodium.

Schéma 2

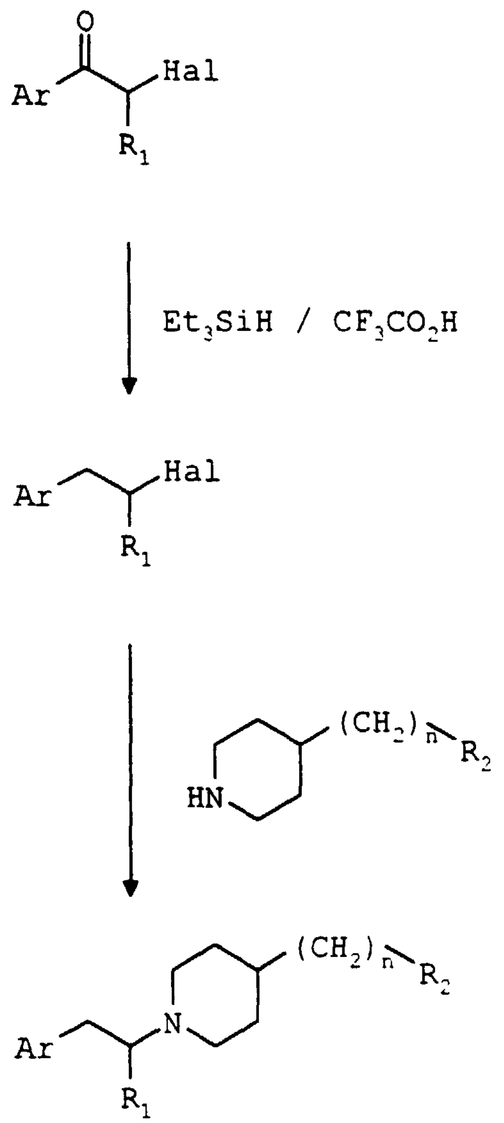


(II)

(IV)

(III)

(Ic)

Les composés de départ de formule générale (II) sont disponibles dans le commerce ou bien sont décrits dans les demandes de brevets EP-0109317, EP-0281309, EP-0351282 et FR-2684379.

Le composé de formule générale (III) dans laquelle n=2 et $R_2$ représente un groupe cyclohexyle est décrit dans *J. Org. Chem.* (1957) **22** 1376 et dans les brevets US-4005093 et US-4028366. Les composés de formule générale (III) dans laquelle n=1 ou 3 et $R_2$ représente un groupe cyclohexyle sont mentionnés, mais non décrits, dans le brevet US-4261891.

Les autres composés de formule générale (III) sont nouveaux et font partie de l'invention à titre d'intermédiaires nécessaires dans le procédé de préparation des composés de formule générale (I).

Les composés de formule générale (III), dans laquelle n = 0, 1 ou 3 et $R_2$ représente un groupe cyclohexyle peuvent être obtenus par hydrogénation catalytique, par exemple en présence de charbon rhodié, des dérivés analogues où $R_2$ représente un groupe phényle.

Les autres composés de formule générale (III) peuvent être obtenus soit par une réaction de Wittig entre le pyridine-4-carboxaldéhyde et un ylure phosphoré obtenu à partir d'un halogénure de cycloalkyle, suivie d'une réduction

par hydrogénolyse totale de l'intermédiaire, soit par alkylation de la 4-méthylpyridine selon la méthode décrite dans le brevet US-3914227, suivie d'une réduction de la cycloalkylalkylpyridine intermédiaire par hydrogénation catalytique en présence d'oxyde de platine.

Les exemples suivants illustrent en détail la préparation de quelques composés selon l'invention. Les microanalyses élémentaires et les spectres IR et RMN confirment les structures des composés obtenus.

Les numéros des composés indiqués entre parenthèses dans les titres correspondent à ceux des tableaux A et B donnés plus loin.

Exemple 1 (Composé N°16A).

1-(4-Chlorophényl)-2-[4-[2-(cyclohexyl)éthyl]pipéridin-1-yl]éthanone.

1.1. 4-[2-(Cyclohexyl)éthyl]pipéridine, chlorhydrate.

On introduit dans un flacon de Parr 20 g (0,088 mole) de chlorhydrate de 4-(2-phényléthyl)pipéridine, 2 g de rhodium sur charbon à 5% et 200 ml d'acide chlorhydrique N, et on effectue une hydrogénation sous une pression d'environ 0,35 MPa à 50°C pendant 15h.

On sépare le catalyseur par filtration, on évapore le filtrat, on le sèche par entraînement avec un mélange d'éthanol et de toluène, et on obtient 17 g de produit cristallin blanc qu'on utilise tel quel dans l'étape suivante.

1.2. 1-(4-Chlorophényl)-2-[4-[2-(cyclohexyl)éthyl]pipéridin-1-yl]éthanone.

On agite un mélange de 8 g (0,0343 mole) de 2-bromo-1-(4-chlorophényl)éthanone, 6,7 g (0,0343 mole) de 4-[2-(cyclohexyl)éthyl]pipéridine, 30 g de carbonate de potassium et 150 ml d'acétonitrile à température ambiante pendant 8h, et on le laisse au repos pendant une nuit.

On le verse dans de l'eau, on sépare l'insoluble par filtration et on le sèche. On obtient 10,1 g de composé. Point de fusion : 71-72°C.

Exemple 2 (Composé N°1A)

(±)-α-(4-Chlorophényl)-4-[2-(cyclohexyl)éthyl]pipéridine-1-éthanol, chlorhydrate.

On introduit dans un ballon 10,1 g (0,029 mole) de 1-(4-chlorophényl)-2-[4-[2-(cyclohexyl)éthyl]pipéridin-1-yl] éthanone, 300 ml de méthanol et 30 ml d'eau, on tiédit le mélange pour obtenir la dissolution, on ajoute, par petites portions, 5 g de borohydrure de potassium et on agite le mélange à température ambiante pendant 6h.

On ajoute de l'eau, puis de l'acide chlorhydrique 3N et on laisse reposer pendant une nuit.

On recueille le solide par filtration, on le lave à l'acétone et on le recristallise dans le propan-2-ol.

On obtient 4,4 g de chlorhydrate.

Point de fusion : 245°C (décomposition).

Exemple 3 (Composé N°4A).

(±)-α-(4-Chlorophényl)-4-[(cyclohexyl)méthyl]-β-méthylpipéridine-1-éthanol, chlorhydrate, isomère érythro.

3.1. 4-[(Cyclohexyl)méthyl]pipéridine, chlorhydrate.

Dans un autoclave de 1 1 on introduit 50 g (0,285 mole) de 4-(phénylméthyl)pipéridine, 500 ml de méthanol, 30 ml d'acide chlorhydrique concentré et 5 g de rhodium sur charbon à 5%, et on effectue un hydrogénation sous environ 7 MPa à 80°C pendant 6h.

On laisse refoidir le mélange, on sépare le catalyseur par filtration et on évapore le filtrat sous pression réduite. On lave le résidu avec de l'acétone et on le sèche.

On obtient 49 g de composé cristallin blanc qu'on utilise tel quel dans l'étape suivante.

Point de fusion : 303-305°C.

3.2. (±)-1-(4-Chlorophényl)-2-[4-[(cyclohexyl)méthyl]pipéridin-1-yl]propanone.

On agite un mélange de 9 g (0,0364 mole) de 2-bromo-1-(4-chlorophényl)propanone, 6,5 g (0,0359 mole) de 4-[(cyclohexyl)méthyl]pipéridine, 30 g de carbonate de potassium et 150 ml d'acétonitrile à température ambiante

pendant 8h, et on laisse reposer le mélange pendant nuit. On le reprend avec de l'eau, on recueille l'insoluble par filtration, on le sèche et on l'utilise tel quel dans l'étape suivante.

3.3. (+)-α-(4-Chlorophényl)-4-[(cyclohexyl)méthyl]-β-méthylpipéridine-1-éthanol, chlorhydrate, isomère érythro.

On introduit dans un ballon 6 g (0,0172 mole) de (±)-1-(4-chlorophényl)-2-[4-[(cyclohexyl)méthyl]pipéridin-1-yl]propanone, 200 ml de méthanol, 25 ml d'acide acétique et 20 ml d'eau, on ajoute, par petites portions, 4 g de borohydrure de potassium, et on laisse reposer pendant une nuit.

On ajoute de l'eau puis de l'acide chlorhydrique, on évapore une partie du solvant, on essore les cristaux formés, on les lave à l'eau, on les recristallise dans le propan-2-ol et on les sèche.

On obtient 4,7 g de chlorhydrate.

Point de fusion : 262-264°C.

Exemple 4 (Composé N°5A).

(±)-α-(4-Chlorophényl)-4-[(cyclohexyl)méthyl]-β-méthylpipéridine-1-éthanol, chlorhydrate, isomère thréo.

On introduit dans un ballon 6 g (0,0172 mole) de (±)-1-(4-chlorophényl)-2-[4-[(cyclohexyl)méthyl]pipéridin-1-yl]propanone, 200 ml de méthanol, du tétrahydrofurane jusqu'à obtenir une solution, puis 20 ml d'eau, on ajoute, par petites portions, 4 g de borohydrure de potassium et on agite le mélange à température ambiante pendant 5h. On ajoute de l'eau, on sépare l'insoluble par filtration et on le purifie par chromatographie sur colonne de gel de silice en éluant avec un mélange de dichlorométhane et d'acétone 97/3.

Après évaporation des fractions les moins polaires et recristallisation dans l'éthanol on obtient 2,3 g de composé.

Point de fusion : 118-119°C.

Exemple 5 (Composé N°8A)

(±)-α-(4-Chlorophényl)-4-(cyclohexyl)pipéridine-1-éthanol.

5.1. Chlorhydrate de 4-(cyclohexyl)pipéridine.

On introduit dans un appareil de Parr 10 g (0,0645 mole) de 4-phénylpyridine, 1 g de charbon rhodié à 5%, 100 ml d'acide chlorhydrique 1N et 10 ml d'acide chlorhydrique concentré, et on effectue une hydrogénation sous 0,35 MPa à 50°C pendant 13 jours (en ajoutant 0,5 g de catalyseur après la première semaine).

On sépare le catalyseur par filtration, on ajoute au filtrat quelques gouttes d'acide chlorhydrique concentré, on le concentre par évaporation, et on effectue deux entraînements à l'éthanol. Après séchage on obtient 11,8 g de solide blanc.

Point de fusion : 310-311°C.

5.2. (±)-α-(4-Chlorophényl)-4-(cyclohexyl)pipéridine-1-éthanol.

On prépare une suspension de 2,03g (0,01 mole) de chlorhydrate de 4-(cyclohexyl)pipéridine, 2,33 g (0,01 mole) de 2-bromo-1-(4-chlorophényl)éthanone, 2,12 g de carbonate de sodium, 40 ml d'éthanol et 10 ml d'eau et on la chauffe au reflux pendant 1h.

On refroidit le mélange, on ajoute 4,4 g de borohydrure de potassium et on l'agite pendant 2 jours à température ambiante.

On ajoute de l'eau, on recueille le précipité par filtration, on le lave à l'eau et on le sèche.

On obtient 2,88 g de composé.

Point de fusion : 135-136°C.

Exemple 6 (Composé N°9A)

(±)-α-(4-Chlorophényl)-4-[3-(cyclohexyl)propyl]pipéridine-1-éthanol.

6.1. Chlorhydrate de 4-[3-(cyclohexyl)propyl]pipéridine.

On introduit dans un flacon de Parr 20,3 g (0,1 mole) de 4-(3-phénylpropyl)pipéridine, 2 g de charbon rhodié

à 5% et 200 ml d'acide chlorhydrique 1N et on effectue une hydrogénation sous 0,35 MPa à 50°C pendant 40h.

On sépare le catalyseur par filtration, on évapore le filtrat, on reprend le résidu avec un mélange de toluène et d'éthanol, on l'évapore de nouveau, on dissout le résidu blanc cristallisé dans 50 ml de propan-2-ol chaud à 5% d'acide chlorhydrique, on refroidit la solution, on ajoute 50 ml d'éther diéthylique, on agite la suspension, on recueille le précipité blanc par filtration, on le lave à l'éther diéthylique et on le sèche.

On obtient 15,86 g de chlorhydrate.

Point de fusion : 229°C.

6.2. (±)-α-(4-Chlorophényl)-4-[3-(cyclohexyl)propyl]pipéridine-1-éthanol.

On prépare une suspension de 1,84 g (0,0075 mole) de chlorhydrate de 4-[3-(cyclohexyl)propyl]pipéridine, 1,75 g (0,0075 mole) de 2-bromo-1-(4-chlorophényl)éthanone, 1,6 g de carbonate de sodium, 40 ml d'éthanol et 10 ml d'eau et on la chauffe au reflux pendant lh30.

On refroidit le mélange, on ajoute 3,59 g de borohydrure de potassium et on l'agite pendant une nuit à température ambiante.

On ajoute 10 ml d'eau, on recueille le précipité par filtration, on le lave à l'eau et on le sèche en présence de pentoxyde de phosphore.

On obtient 2,45 g de composé.

Point de fusion : 85-86°C.

Exemple 7 (Composé N°12A)

1-[2-(4-chlorophényl)éthyl]-4-[(cyclohexyl)méthyl]pipéridine, chlorhydrate.

On introduit dans un ballon 3,0 g (0,0089 mole) de α-(4-chlorophényl)-4-[(cyclohexyl)méthyl]pipéridine-1-éthanol en suspension dans 25 ml de toluène, on refroidit la suspension par un bain de glace, on ajoute une solution de 3 ml de chlorure de thionyle dans 10 ml de toluène, et on agite le mélange à température ambiante pendant 5h30.

On évapore le solvant sous pression réduite, on lave le résidu avec du toluène pour éliminer toute trace de chlorure de thionyle, on évapore le solvant, on reprend le résidu avec 60 ml de tétrahydrofurane et on verse la suspension, goutte à goutte, dans une suspension de 0,7 g d'hydrure de lithium et d'aluminium dans 15 ml de tétrahydrofurane et, tout en agitant le mélange qui s'épaissit, on le chauffe progressivement et on le maintient au reflux pendant 1h30.

On laisse refroidir, on reprend le mélange avec 40 ml d'eau et 30 ml d'acétate d'éthyle, on sépare la phase organique et on évapore le solvant sous pression réduite.

On purifie le résidu par chromatographie sur colonne de gel de silice en éluant avec un mélange de dichlorométhane et de méthanol 95/5.

On prépare le chlorhydrate dans le propanol et, après recristallisation dans 30 ml de propan-2-ol, on isole finalement 0,77 g de composé.

Point de fusion : 284-285°C.

Exemple 8 (Composé N°14A)

1-[2-(4-Chlorophényl)éthyl]-4-[2-(cyclohexyl)éthyl]pipéridine, chlorhydrate

On introduit dans un ballon 3,0 g (0,0085 mole) de (±)-α-(4-chlorophényl)-4-[2-(cyclohexyl)éthyl]pipéridine-1-éthanol en suspension dans 25 ml de toluène, on refroidit la suspension par un bain de glace, on ajoute une solution de 3 ml de chlorure de thionyle dans 10 ml de toluène, et on agite le mélange à température ambiante pendant 5h30.

On évapore le solvant sous pression réduite, on lave le résidu avec du toluène pour éliminer toute trace de chlorure de thionyle, on évapore le solvant, on reprend le résidu avec 60 ml de tétrahydrofurane et on verse la suspension, goutte à goutte, dans une suspension de 0,7 g d'hydrure de lithium et d'aluminium dans 10 ml de tétrahydrofurane et, tout en agitant le mélange, on le chauffe au reflux pendant 1h30, puis on le laisse reposer une nuit.

On le reprend avec 40 ml d'eau et 30 ml d'acétate d'éthyle, on sépare la phase organique et on évapore le solvant sous pression réduite.

On purifie le résidu par chromatographie sur colonne de gel de silice en éluant avec un mélange de dichlorométhane et de méthanol 95/5.

On prépare le chlorhydrate en dissolvant la base dans l'éthanol et en ajoutant de l'acide chlorhydrique jusqu'à pH=1, et, après recristallisation dans le propan-2-ol, on isole finalement 0,30 g de composé.

Point de fusion : 290-291°C.

Exemple 9 (Composé N°7A)

(±)-α-(4-Chlorophényl)-4-[(cyclopentyl)méthyl]pipéridine-1-éthanol.

9.1. Chlorhydrate de 4-[(cyclopentyl)méthyl]pipéridine (1ère variante).

9.1.1. 4-[(Cyclopentyl)méthyl]pyridine.

Dans un ballon tricol de 1 1 on condense 300 ml d'ammoniac à -78°C, on ajoute 100 ml d'éther diéthylique et, à -78°C, on ajoute, par petites portions, 8,28 g (0,36 mole) de sodium. On agite le mélange bleu intense pendant 15 min, et on ajoute, goutte à goutte, 30 g (0,32 mole) de 4-méthylpyridine. La coloration passe du bleu au jaune. On ajoute ensuite, toujours à -78°C, 47,68 g (0,32 mole) de bromocyclopentane en solution dans 100 ml d'éther diéthylique, et on maintient l'agitation à -78°C pendant 2h.

On ajoute du chlorure d'ammonium, et on évapore l'ammoniac et l'éther grâce à un bain d'eau tiède.

On reprend le résidu avec 200 ml d'éther diéthylique et 100 ml d'eau, et on effectue trois extractions à l'éther. On lave la phase organique deux fois à l'eau, on la sèche sur sulfate de magnésium, on la filtre et on évapore le solvant sous pression réduite. On obtient 10 g de produit brut huileux qu'on distille sous pression réduite.

On obtient 2 g de produit qu'on utilise tel quel dans l'étape suivante.

9.1.2. Chlorhydrate de 4-[(cyclopentyl)méthyl]pipéridine.

On introduit dans un flacon de Parr une solution de 1,6 g de 4-[(cyclopentyl)méthyl]pyridine dans 80 ml d'un mélange 1/1 d'éthanol et d'acide chlorhydrique 1N et 300 mg d'oxyde de platine, et on effectue une hydrogénation sous 0,35 MPa à 25°C.

On sépare le catalyseur par filtration, on évapore les solvants sous pression réduite, on ajoute au résidu une solution aqueuse saturée d'hydrogénocarbonate de sodium, et on extrait le mélange trois fois avec de l'acétate d'éthyle. On lave la phase organique deux fois à l'eau et une fois avec une solution aqueuse saturée de chlorure de sodium, on la sèche sur sulfate de magnésium, on la filtre et on évapore le solvant sous pression réduite.

On obtient 1,6 g de produit qu'on purifie par chromatographie sur colonne de gel de silice en éluant avec un mélange 95/5 de méthanol et d'ammoniaque à 25%.

On obtient 1,6 g de produit purifié sous forme de base libre. On prépare le chlorhydrate par dissolution de la base dans une solution 0,1N d'acide chlorhydrique dans le propan-2-ol, puis recristallisation dans un mélange d'acétate d'éthyle et de méthanol.

Point de fusion : 275°C.

9.2. Chlorhydrate de 4-[(cyclopentyl)méthyl]pipéridine (2ème variante).

9.2.1. Bromure de cyclopentyltriphénylphosphonium.

On chauffe un mélange de 17,57 g (0,067 mole) de triphénylphosphine et 10 g (0,067 mole) de bromocyclopentane à 200°C (température du bain) sous atmosphère inerte pendant 2h. On refroidit le mélange, on ajoute du benzène, on agite, on collecte le précipité formé par filtration et on le recristallise dans un mélange de d'acétate d'éthyle et de méthanol.

On obtient 16 g de produit.

Point de fusion : 260°C.

9.2.2. 4-[(Cyclopentylidène)méthyl]pyridine.

Sous atmosphère inerte on introduit dans un ballon bicol de 250 ml une suspension de 8,88 g (0,022 mole) de bromure de cyclopentyltriphénylphosphonium dans 100 ml de tétrahydrofurane, on la refroidit à -78°C, on ajoute, goutte à goutte, 13,75 ml d'une solution 1,6M de butyllithium dans l'hexane, on agite le mélange, devenu rouge, pendant 30 min, puis on ajoute, goutte à goutte, 2,359 g (0,022 mole) de pyridine-4-carboxaldéhyde dissous dans 50 ml de tétrahydrofurane, et on agite le mélange pendant 1h à -78°C puis pendant 3h à température ambiante.

On ajoute 50 ml d'eau et 100 ml d'acétate d'éthyle, on sépare la phase aqueuse, on l'extrait trois fois à l'acétate d'éthyle, on lave la phase organique deux fois à l'eau et une fois avec une solution saturée de chlorure de sodium, on la sèche sur sulfate de magnésium, on la filtre et on évapore le solvant sous pression réduite.

On obtient 10 g de produit brut qui, après purification par chromatographie sur colonne de gel de silice,

donne 2,69 g de composé.

9.2.3. Chlorhydrate de 4-[(cyclopentyl)méthyl]pipéridine.

On introduit dans un flacon de Parr une solution de 4,815 g (0,030 mole) de 4-[(cyclopentylidène)méthyl] pyridine dans 180 ml d'un mélange 1/1 d'éthanol et d'acide chlorhydrique 1N, on ajoute 2,5 g de catalyseur d'Adams ($PtO_2$), et on effectue une hydrogénation à 25°C sous environ 0,35 MPa.

On sépare le catalyseur par filtration, on concentre le filtrat sous pression réduite, on ajoute au résidu une solution aqueuse saturée d'hydrogénocarbonate de sodium, on extrait le mélange trois fois à l'acétate d'éthyle, on lave la phase organique deux fois à l'eau et une fois avec une solution aqueuse saturée de chlorure de sodium, on la sèche sur sulfate de magnésium, on la filtre et on évapore le solvant sous pression réduite.

On obtient 5 g de produit brut qu'on purifie par chromatographie sur colonne de gel de silice en éluant avec un mélange 95/5 de méthanol et d'ammoniaque à 25%.

On obtient 4,9 g de base pure dont on forme le chlorhydrate dans une solution 0,1N d'acide chlorhydrique dans le propan-2-ol.

Point de fusion : 275-276°C.

9.3. (±)-α-(4-Chlorophényl)-4-[(cyclopentyl)méthyl]pipéridine-1-éthanol.

Dans un ballon de 100 ml on introduit 0,57 g (0,0024 mole) de 2-bromo-1-(4-chlorophényl)éthanone, 500 mg (0,0024 mole) de chlorhydrate de 4-[(cyclopentyl)méthyl]pipéridine, 0,52 g (0,0049 mole) de carbonate de sodium, 13 ml d'éthanol et 3 ml d'eau, et on chauffe le mélange à 80°C (température du bain) pendant 1h30.

On refroidit le mélange, on ajoute 1 g de borohydrure de potassium, on agite le mélange et on l'abandonne pendant une nuit.

On ajoute 26 ml d'eau, on agite la suspension, on sépare le solide par filtration, on le lave à l'eau, on le sèche en présence de pentoxyde de phosphore.

On obtient 0,7 g de produit qu'on purifie par chromatographie sur colonne de gel de silice en éluant avec un mélange de dichlorométhane et de méthanol 95/5.

Après recristallisation et séchage on isole finalement 0,366 g de composé.

Point de fusion : 104-105°C.

<u>Exemple 10</u> (Composé N°3A)

(±)-α-(4-Chlorophényl)-4-[(cyclohexyl)méthyl]pipéridine-1-éthanol.

Dans un ballon de 500 ml on introduit 7 g (0,03 mole) de 2-bromo-1-(4-chlorophényl)éthanone, 5,44 g (0,03 mole) de 4-[(cyclohexyl)méthyl]pipéridine, 3,18 g (0,03 mole) de carbonate de sodium, 160 ml d'éthanol et 40 ml d'eau, et on chauffe le mélange au reflux pendant 2h.

On le laisse revenir à température ambiante, on ajoute 10 g de borohydrure de potassium, on agite à température ambiante pendant 30 mn et on laisse reposer pendant une nuit.

On verse le mélange dans 320 ml d'eau, on sépare le précipité jaune par filtration et on le sèche en présence de pentoxyde de phosphore. On obtient 8,8 g de produit brut qu'on purifie par chromatographie sur colonne de gel de silice en éluant avec un mélange de dichlorométhane et de méthanol 90/10. On obtient 6,36 g de composé qu'on recristallise dans l'éthanol.

Point de fusion : 122-123°C.

<u>Exemple 11</u> (Composé N°10A)

(+)-α-(4-Chlorophényl)-4-[(cyclohexyl)méthyl]pipéridine-1-éthanol.

Dans un ballon de 500 ml, sous atmosphère d'azote, on introduit 2,8 g (0,0083 mole) de (±)-α-(4-chlorophényl)-4-[(cyclohexyl)méthyl]pipéridine-1-éthanol, 2,37 g (0,01 mole) de (-)-tartrate de diisopropyle et 80 ml de dichlorométhane, et on ajoute 4,95 g, soit 5,14 ml (0,0174 mole) de tétraisopropoxyde de titane ; la coloration du mélange vire au jaune. On le refroidit à -25°C avec un bain d'acétone et de glace carbonique, et on ajoute 1,53 ml d'une solution 3,3M d'hydroperoxyde de tertiobutyle dans du toluène, et on poursuit l'agitation à -25°C pendant 2h30.

On ajoute 80 ml d'éther diéthylique, 4 ml d'eau et 5 ml de soude à 30%, on laisse le mélange revenir à température ambiante tout en l'agitant vivement, et on le laisse reposer pendant une nuit.

On élimine le précipité blanc (sels de titane) par filtration, et on évapore le filtrat. On obtient 1,67 g de solide

cristallisé blanc qu'on purifie par chromatographie sur colonne de gel de silice en éluant avec de l'acétate d'éthyle. On obtient 0,53 g de cristaux blancs qu'on recristallise deux fois dans l'éthanol, pour isoler finalement 0,21 g d'énantiomère dextrogyre (ee=92,6%).

Point de fusion : 140-141°C $[\alpha]_D^{20}$ = +46,0° (c=1 ; $CHCl_3$).

Exemple 12 (Composé N°11A)

(-)-α-(4-Chlorophényl)-4-[(cyclohexyl)méthyl]pipéridine-1-éthanol.

On procède comme décrit dans l'exemple précédent, en utilisant du tartrate de diisopropyle dextrogyre au lieu de lévogyre. A partir de 3,36 g (0,01 mole) de racémate on isole finalement 0,38 g d'énantiomère lévogyre (ee=90,5%).

Point de fusion : 140-141°C $[\alpha]_D^{20}$ = -46,0° (c=1 ; $CHCl_3$).

Exemple 13 (Composé N°18A)

(±)-α-(4-Chlorophényl)-4-[(cycloheptyl)méthyl]pipéridine-1-éthanol.

13.1. Chlorhydrate de 4-[(cycloheptyl)méthyl]pipéridine.

On utilise le procédé (2ème variante) décrit à propos du chlorhydrate de 4-[(cyclopentyl)méthyl]pipéridine, à partir de bromocycloheptane au lieu de bromocyclopentane.

Point de fusion : 260°C.

13.2. (±)-α-(4-Chlorophényl)-4-[(cycloheptyl)méthyl]pipéridine-1-éthanol.

On agite un mélange de 0,4 g (0,00171 mole) de 2-bromo-1-(4-chlorophényl)éthanone, 0,4 g (0,00171 mole) de chlorhydrate de 4-[(cycloheptyl)méthyl]pipéridine et 0,4 g de carbonate de sodium dans 20 ml d'éthanol et 5 ml d'eau, et on chauffe le mélange au reflux pendant 2h.

On le refroidit, on ajoute 0,8 g de borohydrure de potassium et on maintient l'agitation à température ambiante pendant une nuit.

On ajoute 40 ml d'eau, on extrait la phase aqueuse à l'acétate d'éthyle, on lave la phase organique à l'eau puis avec une solution aqueuse saturée de chlorure de sodium, on la sèche sur sulfate de sodium et on évapore le solvant sous pression réduite. On purifie le résidu par chromatographie sur colonne de gel de silice en éluant avec un mélange 95/5 de dichlorométhane et de méthanol. Après recristallisation dans l'éthanol on isole finalement 0,097 g de composé.

Point de fusion : 116-117°C.

Exemple 14 (Composé N°9B)

5-[2-[4-(2-Cyclohexyléthyl)pipéridin-1-yl]-1-oxoéthyl]-3*H*-indol-2-one.

Dans un ballon de 500 ml on introduit 3,14 g (0,015 mole) de 5-(chloroacétyl)-3*H*-indol-2-one, 3,47 g (0,015 mole) de chlorhydrate de 4-(2-cyclohexyléthyl)pipéridine, 3,17 g (0,03 mole) de carbonate de sodium, 80 ml d'éthanol et 20 ml d'eau, et on chauffe le mélange au reflux pendant 1h30.

On le laisse refroidir, on le verse dans 200 ml d'eau, on sépare le précipité par filtration, on le lave à l'éther diéthylique, on le sèche en présence de pentoxyde de phosphore.

On obtient 4 g de produit brut qu'on purifie par chromatographie sur colonne de gel de silice en éluant avec un mélange de dichlorométhane et de méthanol 95/5, puis par recristallisation dans l'éthanol.

Après lavage à l'éther diéthylique et séchage en présence de pentoxyde de phosphore on isole finalement 0,96 g de composé.

Point de fusion : 180-181°C.

Exemple 15 (Composé N°4B)

(±)-5-[2-[4-(2-Cyclohexyléthyl)pipéridin-1-yl]-1-hydroxyéthyl]-3*H*-indol-2-one.

Dans un ballon de 500 ml on introduit 3,14 g (0,015 mole) de 5-(chloroacétyl)-3*H*-indol-2-one, 3,47 g (0,015

mole) de chlorhydrate de 4-(2-cyclohexyléthyl)pipéridine, 3,18 g (0,03 mole) de carbonate de sodium, 100 ml d'éthanol et 20 ml d'eau, et on chauffe le mélange à 120°C (température du bain) pendant 1h30.

On le refroidit dans un bain de glace, on ajoute 6,61 g de borohydrure de potassium, et on agite le mélange à température ambiante pendant une nuit.

On verse le mélange dans 200 ml d'eau, on l'agite pendant 10 min, on sépare le précipité par filtration, on le lave à l'eau et à l'éther de pétrole et on le sèche en présence de pentoxyde de phosphore.

On obtient 4,34 g de produit brut qu'on purifie par chromatographie sur colonne de gel de silice en éluant avec un mélange de dichlorométhane et de méthanol 90/10.

Après recristallisation et séchage on isole finalement 1,48 g de composé.

Point de fusion : 210-211°C.

Exemple 16 (Composé N°8B)

5-[2-[4-(2-Cyclohexyléthyl)pipéridin-1-yl]éthyl]-3*H*-indol-2-one.

16.1. 5-(2-Chloroéthyl)-3*H*-indol-2-one.

Dans un ballon de 500 ml on introduit 15 g (0,0715 mole) de 5-(chloroacétyl)-3H-indol-2-one en suspension dans 60 ml d'acide trifluoroacétique, on refroidit le mélange avec un bain de glace, on ajoute, goutte à goutte, 26,13 ml (0,164 mole) de triéthylsilane, on laisse le mélange revenir à température ambiante et on maintient l'agitation pendant une nuit.

On verse le mélange dans 300 ml d'eau glacée, on l'agite pendant 15 min, on collecte le précipité beige par filtration, on le lave à l'eau et à l'hexane, et on le sèche en présence de pentoxyde de phosphore.

On obtient 13,75 g de composés qu'on utilise tel quel dans l'étape suivante.

16.2. 5-[2-[4-(2-Cyclohexyléthyl)pipéridin-1-yl]éthyl]-3*H*-indol-2-one.

Dans un ballon de 500 ml on introduit 2,9 g (0,015 mole) de 5-(2-chloroéthyl)-3*H*-indol-2-one, 3,47 g (0,015 mole) de chlorhydrate de 4-(2-cyclohexyléthyl)pipéridine, 3,17 g (0,03 mole) de carbonate de sodium, 50 ml de *N,N*-diméthylformamide, et on chauffe le mélange à 120°C (température du bain) pendant 5h.

On ajoute 150 ml d'eau, on agite pendant 30 min, on sépare le précipité marron par filtration, et on le sèche en présence de pentoxyde de phosphore.

On obtient 4,56 g de produit brut qu'on purifie par chromatographie sur colonne de gel de silice en éluant avec un mélange de dichlorométhane et de méthanol 90/10.

Après recristallisation dans le propan-2-ol, lavage à l'éther diéthylique et séchage en présence de pentoxyde de phosphore on isole finalement 0,85 g de composé.

Point de fusion : 190-191°C.

Exemple 17 (Composé N°7B)

6-[2-[4-(2-Cyclohexyléthyl)pipéridin-1-yl]-1-oxoéthyl]-3,4-dihydro-1*H*-quinoléin-2-one.

Dans un ballon de 500 ml on introduit 3,35 g (0,015 mole) de 6-(chloroacétyl)-3,4-dihydro-1*H*-quinoléin-2-one, 3,47 g (0,015 mole) de chlorhydrate de 4-(2-cyclohexyléthyl)pipéridine, 3,17 g (0,03 mole) de carbonate de sodium, 80 ml d'éthanol et 20 ml d'eau, et on chauffe le mélange au reflux pendant 1h30.

On le laisse refroidir, on le verse dans 200 ml d'eau, on l'agite pendant 15 min, on sépare le précipité par filtration, on le lave à l'eau et on le sèche en présence de pentoxyde de phosphore.

On obtient 5,4 g de produit brut qu'on purifie par chromatographie sur colonne de gel de silice en éluant avec un mélange de dichlorométhane et de méthanol 90/10, puis par recristallisation dans l'éthanol.

Après lavage à l'éther diéthylique et séchage en présence de pentoxyde de phosphore on isole finalement 3,82 g de composé. Point de fusion : 176-177°C.

Exemple 18 (Composé N°3B)

(±)-6-[2-[4-(2-Cyclohexyléthyl)pipéridin-1-yl]-1-hydroxyéthyl]-8-fluoro-3,4-dihydro-1*H*-quinoléin-2-one.

Dans un ballon de 500 ml on introduit 3,62 g (0,015 mole) de 6-(chloroacétyl)-8-fluoro-3,4-dihydro-1*H*-quinoléin-2-one, 3,47 g (0,015 mole) de chlorhydrate de 4-(2-cyclohexyléthyl)pipéridine, 3,18 g (0,03 mole) de carbonate

de sodium, 80 ml d'éthanol et 20 ml d'eau, et on chauffe le mélange au reflux pendant 1h30.

On le laisse refroidir, on ajoute 6,5 g de borohydrure de potassium, et on agite le mélange à température ambiante pendant une nuit.

On ajoute 160 ml d'eau, on agite pendant 15 min, on sépare le précipité beige par filtration, on le lave à l'eau et à l'éther de pétrole et on le sèche en présence de pentoxyde de phosphore.

On obtient 5 g de produit brut qu'on purifie par chromatographie sur colonne de gel de silice en éluant avec un mélange de dichlorométhane et de méthanol 90/10.

Après recristallisation et séchage en présence de pentoxyde de phosphore on isole finalement 2,7 g de composé.

Point de fusion : 154-155°C.

Exemple 19 (Composé N°5B)

6-[2-[4-(2-Cyclohexyléthyl)pipéridin-1-yl]éthyl]-3,4-dihydro-1*H*-quinoléin-2-one.

19.1. 6-(2-Chloroéthyl)3,4-dihydro-1*H*-quinoléin-2-one.

On introduit dans un ballon 5,36 g (0,024 mole) de 6-(chloroacétyl)-3,4-dihydro-1*H*-quinoléin-2-one et 18,5 ml d'acide trifluoroacétique, on refroidit la suspension dans un bain de glace, et on ajoute, goutte à goutte, 8,77 ml (0,055 mole) de triéthylsilane, et on agite le mélange à température ambiante pendant 16h.

On le verse dans 100 ml d'eau glacée, on l'agite pendant 15 min, on sépare le précipité par filtration, on le lave à l'eau puis à l'hexane, et on le sèche en présence de pentoxyde de phosphore.

On obtient 5 g de composé.

Point de fusion : 163-164°C.

19.2. 6-[2-[4-(2-Cyclohexyléthyl)pipéridin-1-yl]éthyl]-3,4-dihydro-1*H*-quinoléin-2-one.

Dans un ballon de 500 ml on introduit 3,14 g (0,015 mole) de 6-(2-chloroéthyl)-3,4-dihydro-1*H*-quinoléin-2-one, 3,47 g (0,015 mole) de chlorhydrate de 4-(2-cyclohexyléthyl)pipéridine, 3,18 g (0,03 mole) de carbonate de sodium, 50 ml de *N,N*-diméthylformamide, et on chauffe le mélange à 120°C (température du bain) pendant 4h30.

On ajoute 150 ml d'eau, on agite pendant 30 min dans un bain de glace, on sépare le précipité par filtration, on le lave à l'eau et on le sèche en présence de pentoxyde de phosphore. On obtient 4,70 g de produit brut qu'on purifie par chromatographie sur colonne de gel de silice en éluant avec un mélange de dichlorométhane et de méthanol 90/10.

Après recristallisation dans le propan-2-ol, lavage à l'éther diéthylique et séchage en présence de pentoxyde de phosphore on isole finalement 2,5 g de composé.

Point de fusion : 192-193°C.

Exemple 20 (Composé N°6B)

(±)-7-[2-[4-(2-Cyclohexyléthyl)pipéridin-1-yl]-1-hydroxyéthyl]-1,3,4,5-tétrahydrobenzo[b]azépin-2-one.

Dans un ballon de 500 ml on introduit 3,56 g (0,015 mole) de 7-(chloroacétyl)-1,3,4,5-tétrahydrobenzo[*b*]azépin-2-one, 3,47 g (0,015 mole) de chlorhydrate de 4-(2-cyclohexyléthyl)pipéridine, 3,18 g (0,03 mole) de carbonate de sodium, 80 ml d'éthanol et 20 ml d'eau, et on chauffe le mélange au reflux pendant 2h.

On le laisse refroidir, on ajoute 6 g de borohydrure de potassium, et on agite le mélange à température ambiante pendant une nuit.

On ajoute 160 ml d'eau, on agite pendant 2h, on sépare le précipité par filtration, on le lave à l'eau et on le sèche en présence de pentoxyde de phosphore.

On obtient 5,1 g de produit brut qu'on purifie par chromatographie sur colonne de gel de silice en éluant avec un mélange de dichlorométhane et de méthanol 90/10.

Après recristallisation dans le propanol et séchage on isole finalement 3 g de composé.

Point de fusion : 188-189°C.

Exemple 21 (Composé N°17B)

6-[2-[4-(3-Cyclohexylpropyl)pipéridin-1-yl]-1-oxoéthyl]-3,4-dihydro-1*H*-quinoléin-2-one.

On introduit dans un ballon 1,0 g (0,0041 mole) de chlorhydrate de 4-[3-(cyclohexyl)propyl]pipéridine, 1,10 g (0,0041 mole) de 6-(bromoacétyl)-3,4-dihydro-1*H*-quinoléin-2-one, 0,87 g (0,0082 mole) de carbonate de sodium, 20 ml d'éthanol et 5,5 ml d'eau, et on chauffe la suspension au reflux pendant 2h30.
On refroidit le mélange à 0°C, on l'agite à cette température pendant 20 min, on ajoute 35 ml d'eau et on maintient l'agitation à 0°C pendant 15 min, on recueille le précipité marron clair par filtration, on lave les cristaux à l'eau et on les sèche en présence de pentoxyde de phosphore pendant une nuit.
Après recristallisation dans le propan-2-ol, lavage à l'eau, lavage à l'hexane, et séchage, on obtient 1,23 g de composé.
Point de fusion : 179°C.

Exemple 22 (Composé N°10B)

(±)-6-[2-[4-(3-Cyclohexylpropyl)pipéridin-1-yl]-1-hydroxyéthyl]-3,4-dihydro-1*H*-quinoléin-2-one.

On introduit dans un ballon 5,5 g (0,0138 mole) de 6-[2-[4-(3-cyclohexylpropyl)pipéridin-1-yl]-1-oxoéthyl]-3,4-dihydro-1*H*-quinoléin-2-one, 80 ml d'éthanol et 20 ml d'acide chlorhydrique 0,5N, on ajoute 6 g de borohydrure de potassium et on agite le mélange à température ambiante pendant une nuit.
On ajoute 160 ml d'eau, on agite pendant 30 min, on sépare le précipité par filtration, on le lave à l'eau et on le sèche. On obtient 5,2 g de produit brut qu'on purifie par chromatographie sur colonne de gel de silice en éluant avec un mélange de dichlorométhane et de méthanol 90/10.
Après recristallisation dans l'éthanol et séchage on isole finalement 4,24 g de composé.
Point de fusion : 151-152°C.

Exemple 23 (Composé N°16B)

6-[2-[4-(Cyclohexyl)pipéridin-1-yl]éthyl]-3,4-dihydro-1*H*-quinoléin-2-one.

On prépare une suspension de 0,565 g (0,00277 mole) de chlorhydrate de 4-(cyclohexyl)pipéridine, 0,70 g (0,00276 mole) de 6-(2-bromoéthyl)-3,4-dihydro-1*H*-quinoléin-2-one, 0,59 g (0,0055 mole) de carbonate de sodium, et 9,5 ml de *N,N*-diméthylformamide, et on chauffe le mélange à 120°C (température du bain) pendant 5h30.
On le laisse refroidir, on ajoute 20 ml d'eau, on agite pendant 15 min, on recueille le solide par filtration, et on lave les cristaux à l'eau.
On obtient 0,73 g de produit qu'on purifie par chromatographie sur colonne de gel de silice en éluant avec un mélange de dichlorométhane et de méthanol 94/6.
Après recristallisation dans 200 ml d'acétone contenant un peu de méthanol, filtration, lavage à l'eau et à l'hexane, puis séchage, on isole finalement 0,466 g de cristaux.
Point de fusion : 212-214°C.

Exemple 24 (Composé N°19B)

6-[2-[4-[(Cyclopentyl)méthyl]pipéridin-1-yl]éthyl]-3,4-dihydro-1*H*-quinoléin-2-one.

On prépare une suspension de 0,30 g (0,00147 mole) de chlorhydrate de 4-[(cyclopentyl)méthyl]pipéridine, 0,375 g (0,00147 mole) de 6-(2-bromoéthyl)-3,4-dihydro-1*H*-quinoléin-2-one, 0,313 g (0,00298 mole) de carbonate de sodium, et 5,5 ml de *N,N*-diméthylformamide, et on chauffe le mélange à 130°C (température du bain) pendant 3h.
On le laisse refroidir, on ajoute 12 ml d'eau, on agite pendant 15 min, on recueille le solide par filtration, et on lave les cristaux avec de l'eau puis avec de l'hexane.
On obtient 0,39 g de produit qu'on purifie par chromatographie sur colonne de gel de silice en éluant avec un mélange de dichlorométhane et de méthanol 94/6.
Après recristallisation dans 60 ml d'acétone contenant un peu de méthanol, filtration, lavage à l'eau et à l'hexane, puis séchage, on isole finalement 0,217 g de cristaux.
Point de fusion : 172-174°C.

Exemple 25 (Composé N°29B)

6-[2-[4-[(Cycloheptyl)méthyl]pipéridin-1-yl]éthyl]-3,4-dihydro-1*H*-quinoléin-2-one.

On introduit dans un ballon une suspension de 0,2 g (0,000864 mole) de chlorhydrate de 4-[(cycloheptyl) méthyl]pipéridine, 3,5 ml de *N,N*-diméthylformamide, 0,22 g (0,000864 mole) de 6-(2-bromoéthyl)-3,4-dihydro-1*H*-quinoléin-2-one, 0,184 g (0,001728 mole) de carbonate de sodium, et on chauffe le mélange au reflux pendant 3h15.

On le refroidit, on le dilue avec 8 ml d'eau, on collecte le précipité par filtration et on le sèche.

On obtient 0,23 g de produit qu'on purifie par chromatographie sur colonne de gel de silice en éluant avec un mélange 96/4 de dichlorométhane et de méthanol.

On isole 0,21 g de solide qu'on recristallise dans 80 ml d'acétone pour obtenir finalement 0,146 g de composé.

Point de fusion : 154-155°C.

Les tableaux A et B qui suivent illustrent les structures chimiques et les propriétés physiques de quelques composés selon l'invention.

Dans les colonnes "$R_2$", "$C_5H_9$" désigne un groupe cyclopentyle, "$C_6H_{11}$" désigne un groupe cyclohexyle et "$C_7H_{13}$" désigne un groupe cycloheptyle.

Dans la colonne "Isom.", "(±)" désigne un racémate, "+" désigne un énantiomère dextrogyre, "-" désigne un énantiomère lévogyre, "/" désigne un composé achiral, "E" désigne les diastéréoisomères érythro et "T" désigne les diastéréoisomères thréo.

Dans la colonne "Sel", "-" désigne un composé à l'état de base, et "HCl" désigne un chlorhydrate.

Dans la colonne "F (°C)", "(d)" désigne un point de fusion avec décomposition.

Tableau A

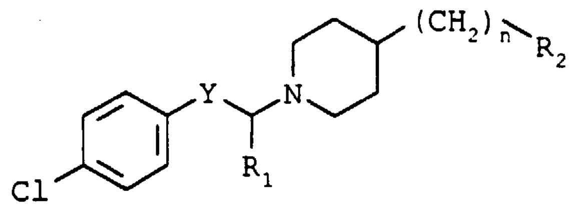

(I)

| N° | Y | $R_1$ | $R_2$ | n | Isom. | Sel | F (°C) |
|---|---|---|---|---|---|---|---|
| 1A | CHOH | H | $C_6H_{11}$ | 2 | (±) | HCl | 245 (d) |
| 2A | CHOH | $CH_3$ | $C_6H_{11}$ | 2 | (±) E | HCl | 230-244 (d) |
| 3A | CHOH | H | $C_6H_{11}$ | 1 | (±) | - | 122-123 |
| 4A | CHOH | $CH_3$ | $C_6H_{11}$ | 1 | (±) E | HCl | 262-264 |
| 5A | CHOH | $CH_3$ | $C_6H_{11}$ | 1 | (±) T | - | 118-119 |
| 6A | CHOH | $CH_3$ | $C_6H_{11}$ | 2 | (±) T | - | 114-115 |
| 7A | CHOH | H | $C_5H_9$ | 1 | (±) | - | 104-105 |
| 8A | CHOH | H | $C_6H_{11}$ | 0 | (±) | - | 135-136 |
| 9A | CHOH | H | $C_6H_{11}$ | 3 | (±) | - | 85-86 |
| 10A | CHOH | H | $C_6H_{11}$ | 1 | (+) | - | 140-141 |
| 11A | CHOH | H | $C_6H_{11}$ | 1 | (-) | - | 140-141 |
| 12A | $CH_2$ | H | $C_6H_{11}$ | 1 | / | HCl | 284-285 |
| 13A | $CH_2$ | H | $C_6H_{11}$ | 0 | / | HCl | 300-301 |
| 14A | $CH_2$ | H | $C_6H_{11}$ | 2 | / | HCl | 290-291 |
| 15A | $CH_2$ | H | $C_6H_{11}$ | 3 | / | HCl | 241-242 |
| 16A | CO | H | $C_6H_{11}$ | 2 | / | - | 71-72 |
| 17A | CO | H | $C_6H_{11}$ | 3 | / | HCl | 210-211 |
| 18A | CHOH | H | $C_7H_{13}$ | 1 | (±) | - | 116-117 |
| 19A | $CH_2$ | H | $C_5H_9$ | 1 | / | HCl | 284-285 |

Note : les pouvoirs rotatoires des composés numéros 10A et 11A sont, respectivement, de +46° et -46° (c=1 ; $CHCl_3$).

Tableau B

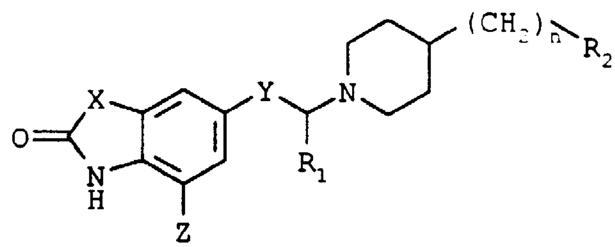

(I)

| N° | X | Y | Z | $R_1$ | $R_2$ | n | Isom. | Sel | F (°C) |
|---|---|---|---|---|---|---|---|---|---|
| 1B | $(CH_2)_2$ | CHOH | H | H | $C_6H_{11}$ | 2 | (±) | - | 192-193 |
| 2B | $(CH_2)_2$ | CHOH | 8-$CH_3$ | H | $C_6H_{11}$ | 2 | (±) | - | 192-193 |
| 3B | $(CH_2)_2$ | CHOH | 8-F | H | $C_6H_{11}$ | 2 | (±) | - | 154-155 |
| 4B | $CH_2$ | CHOH | H | H | $C_6H_{11}$ | 2 | (±) | - | 210-211 |
| 5B | $(CH_2)_2$ | $CH_2$ | H | H | $C_6H_{11}$ | 2 | / | - | 192-193 |
| 6B | $(CH_2)_3$ | CHOH | H | H | $C_6H_{11}$ | 2 | (±) | - | 188-189 |
| 7B | $(CH_2)_2$ | CO | H | H | $C_6H_{11}$ | 2 | / | - | 176-177 |
| 8B | $CH_2$ | $CH_2$ | H | H | $C_6H_{11}$ | 2 | / | - | 190-191 |
| 9B | $CH_2$ | CO | H | H | $C_6H_{11}$ | 2 | / | - | 180-181 |
| 10B | $(CH_2)_2$ | CHOH | H | H | $C_6H_{11}$ | 3 | (±) | - | 151-152 |
| 11B | $(CH_2)_2$ | CO | H | H | $C_6H_{11}$ | 1 | / | - | 191-193 |
| 12B | $(CH_2)_2$ | CHOH | H | H | $C_6H_{11}$ | 1 | (±) | - | 190-192 |
| 13B | $(CH_2)_2$ | $CH_2$ | H | H | $C_6H_{11}$ | 1 | / | - | 169-171 |
| 14B | $(CH_2)_2$ | CO | H | H | $C_6H_{11}$ | 0 | / | - | 190-192 |
| 15B | $(CH_2)_2$ | CHOH | H | H | $C_6H_{11}$ | 0 | (±) | - | 230-232 |
| 16B | $(CH_2)_2$ | $CH_2$ | H | H | $C_6H_{11}$ | 0 | / | - | 212-214 |
| 17B | $(CH_2)_2$ | CO | H | H | $C_6H_{11}$ | 3 | / | - | 179 |
| 18B | $(CH_2)_2$ | $CH_2$ | H | H | $C_6H_{11}$ | 3 | / | - | 150-152 |
| 19B | $(CH_2)_2$ | $CH_2$ | H | H | $C_5H_9$ | 1 | / | - | 172-174 |
| 20B | $CH_2$- | CO | H | H | $C_6H_{11}$ | 1 | / | - | 164-166 |
| 21B | $CH_2$- | CHOH | H | H | $C_6H_{11}$ | 1 | (±) | - | 179-181 |
| 22B | $CH_2$- | $CH_2$ | H | H | $C_6H_{11}$ | 1 | / | - | 160-162 |

| N° | X | Y | Z | $R_1$ | $R_2$ | n | Isom. | Sel | F (°C) |
|---|---|---|---|---|---|---|---|---|---|
| 23B | $CH_2$ | CO | H | H | $C_6H_{11}$ | 0 | / | - | 179-181 |
| 24B | $CH_2$ | CHOH | H | H | $C_6H_{11}$ | 0 | (±) | - | 204-206 |
| 25B | $CH_2$ | CO | H | H | $C_6H_{11}$ | 3 | / | - | 154-156 |
| 26B | $CH_2$ | $CH_2$ | H | H | $C_6H_{11}$ | 0 | / | - | 187-189 |
| 27B | $(CH_2)_2$ | CHOH | H | H | $C_5H_9$ | 1 | (±) | - | 189-191 |
| 28B | $(CH_2)_2$ | CO | H | H | $C_5H_9$ | 1 | / | - | 162-164 |
| 29B | $(CH_2)_2$ | $CH_2$ | H | H | $C_7H_{13}$ | 1 | / | - | 154-155 |
| 30B | $(CH_2)_2$ | CHOH | H | H | $C_7H_{13}$ | 1 | (±) | - | 190-192 |
| 31B | $CH_2$ | $CH_2$ | H | H | $C_6H_{11}$ | 3 | / | - | 154-156 |
| 32B | $CH_2$ | CHOH | H | H | $C_6H_{11}$ | 3 | (±) | - | 164-166 |
| 33B | $CH_2$ | CO | H | H | $C_5H_9$ | 1 | / | - | 157-159 |
| 34B | $CH_2$ | $CH_2$ | H | H | $C_5H_9$ | 1 | / | - | 162-164 |
| 35B | $CH_2$ | CHOH | H | H | $C_5H_9$ | 1 | (±) | - | 173-175 |
| 36B | $CH_2$ | $CH_2$ | H | H | $C_7H_{13}$ | 1 | / | - | 156-158 |

Les composés de l'invention ont fait l'objet d'essais qui ont mis en évidence leur intérêt comme substances thérapeutiques.

Les propriétés neurotrophes des composés de l'invention ont été démontrées *in vivo* par leurs effets sur la régénération du nerf sciatique chez le rat.

Ces effets ont été évalués après lésion par congélation locale du nerf sciatique chez le rat. La lésion par congélation détruit les fibres du nerf sciatique qui subissent une dégénérescence wallérienne au site de la lésion et dans tout le tronçon distal. Ce type de lésion conserve les gaines nerveuses et permet une régénération nerveuse dans des conditions reproductibles. Le processus de régénération commence à partir du côté proximal dans les heures qui suivent la lésion. La vitesse de régénération des fibres sensitives est mesurée par un test de pincement ("pinch-test") 8 jours après la lésion.

Les animaux sont des rats adultes mâles de souche Sprague Dawley (Iffa Credo) pesant 250 g environ. Après anesthésie des animaux par du pentobarbital sodique (60 mg/kg), la peau de la cuisse est désinfectée à l'alcool et incisée au niveau de la jonction des biceps fémoraux. Le nerf sciatique est mis à nu après par écartement des muscles Lateralis et Biceps Femoris. Le point de la lésion est repéré par une microsuture (Ethilon™ noir 10-0) sur le périnèvre au dessus de la trifurcation du nerf sciatique. La lésion du nerf sciatique est effectuée sur 1 mm par 6 cycles de congélation-décongélation à l'aide d'une cryode en cuivre pré-refroidie dans de l'azote liquide. La plaie est ensuite refermée et traitée par un antibiotique (Exoseptoplix®). Les animaux sont mis en cages individuelles et surveillés quotidiennement.

Après l'opération, les animaux sont répartis en plusieurs lots de 6 individus :

- témoins lésés qui reçoivent une injection intrapéritonéale de Tween 80™ à 0,1%, 10 min et 6h après la lésion, puis deux fois par jour, du deuxième au huitième jour,
- animaux lésés traités qui reçoivent une injection intrapéritonéale d'un composé à étudier, administré aux doses de 0,3, 1 ou 3 mg/kg dans du Tween 80™ à 0,1%, 10 min et 6h après la lésion, puis deux fois par jour, du deuxième au huitième jour.

Huit jours après l'opération, les animaux sont légèrement anesthésiés, et le nerf sciatique est remis à nu pour effectuer

le pinch-test. Ce test consiste à pincer légèrement le nerf à l'aide d'un forceps en commençant par la région la plus distale du nerf et en remontant tous les 0,5 mm. Une réponse réflexe (contraction des muscles de la région postérieure de l'animal) est observée là où le front des fibres sensitives régénérées est présent. Ce point est marqué par une microsuture. Le nerf est ensuite prélevé, la distance entre le site de la lésion et la microsuture distale est mesurée sur un papier millimétré sous microscope opératoire. Après prélèvement du nerf les animaux sont sacrifiés par une overdose de pentobarbital.
On constate que, chez les animaux traités, l'administration des composés de l'invention augmente la distance parcourue par les fibres sensitives de plus de 10% par rapport aux témoins.

Les propriétés neurotrophes des composés de l'invention ont également été étudiées *in vivo* par leurs effets sur la régénération du nerf sciatique après lésion par écrasement associée à un traitement par la vincristine.
La vincristine est un alcaloïde de la pervenche utilisé dans le traitement de certains cancers, et dont le mécanisme d'action est bien établi. La vincristine se fixe sur une protéine cytoplasmique, la tubuline, et perturbe sa polymérisation en microtubules. Ces derniers sont des éléments majeurs intervenant dans la régénération nerveuse après une lésion. En efet, les microtubules vont assurer le transport axonal des macromolécules nouvellement synthétisées et devant être acheminées vers la partie distale du nerf, pour assurer la formation et l'élongation des cônes de croissance. Ainsi l'administration de la vincristine perturbe le transport axonal rapide et ralentit, ou inhibe, selon les doses administrées, la régénération du nerf après une lésion.
Modèle expérimental : après anesthésie au pentobarbital, au jour $J_0$, on écrase le nerf sciatique droit des rats pendant 1,5 min. Le lendemain ($J_1$)les animaux reçoivent une administration unique de 0,2 mg/kg de vincristine et, au jour $J_7$, on évalue la vitesse de régénération au moyen du test de pincement. La dose de 0,2 mg/kg de vincristine réduit d'environ 20% la distance de régénération évaluée à $J_7$ chez les animaux témoins, non traités par les composés à tester. Ces derniers sont administrés aux animaux par voie intrapéritonéale, deux fois par jour, de $J_0$ à $J_6$ (10 min et 6 h après la lésion, à $J_0$, puis matin et soir, à 6 h d'intervalle, de $J_1$ à $J_6$).
Les composés les plus actifs dans cet essai augmentent la vitesse de régénération du nerf sciatique lésé, chez les animaux traités à la vincristine, de 12 à 14%.

Les composés de l'invention ont aussi fait l'objet d'un essai d'inhibition de la liaison du [$^3$H]ifenprodil dans le cortex cérébral de rat (Schoemaker et coll., *Eur. J. Pharmacol.* (1990) **183** 1670).
Le rat mâle Sprague-Dawley de 150 à 230 g est sacrifié et le cortex cérébral est homogénéisé dans 20 volumes de tampon Tris-HCl à 50mM (pH=7,4 à 25°C) glacé, au moyen d'un appareil Ultra-Turrax™ (Ikawerk) ou Polytron™ (Kinematica). L'homogénat est lavé deux fois par centrifugation pendant 10 minutes à 45000×g, le culot étant remis en suspension dans du tampon frais. Le culot final est repris dans 20 volumes du même tampon. Un aliquote de 100 µl de cette suspension est incubé dans un volume final de 1000 µl avec 0,5 nM de [$^3$H]ifenprodil (activité spécifique : 30 à 35 Ci/mmole) pendant 30 minutes à 37°C, en l'absence ou en présence de substance compétitrice. Après incubation les membranes sont récupérées par filtration sur des filtres Whatman GF/B™ prétraités avec de la polyéthylèneimine à 0,05%, puis lavés avec deux fois 5 ml de tampon glacé.
On détermine la liaison non spécifique avec de l'ifenprodil 10µM, on analyse les données selon les méthodes usuelles, et on calcule la concentration $CI_{50}$, concentration qui inhibe de 50% la liaison du [$^3$H]ifenprodil.
Les $CI_{50}$ des composés les plus actifs sont inférieures à 25 nM.

Enfin les composés ont été soumis au test de l'ischémie cérébrale globale chez la souris.
L'ischémie est due à un arrêt cardiaque induit par une injection intraveineuse rapide de chlorure de magnésium. Dans ce test on mesure le "temps de survie", c'est-à-dire l'intervalle entre le moment de l'injection de chlorure de magnésium et le dernier mouvement respiratoire observable de chaque souris. Ce dernier mouvement est considéré comme l'indice ultime d'une fonction du système nerveux central.
L'arrêt respiratoire apparaît approximativement 19 secondes après l'injection de chlorure de magnésium.
Des souris mâles (Charles River CD1) sont étudiées par groupes de 10. Elles sont nourries et abreuvées ad libitum avant les essais. Le temps de survie est mesuré 10 minutes après l'administration intrapéritonéale des composés de l'invention. Les résultats sont donnés sous la forme de la différence entre le temps de survie mesuré dans un groupe de 10 souris ayant reçu le composé et le temps de survie mesuré dans un groupe de 10 souris ayant reçu le liquide véhicule.
Les rapports entre les modifications dans le terme de survie et la dose du composé sont enregistrés graphiquement selon une courbe semilogarithmique.
Cette courbe permet le calcul de la "dose efficace 3 secondes" ($DE_{3''}$), c'est-à-dire la dose (en mg/kg) qui produit une augmentation de 3 secondes du temps de survie par rapport au groupe témoin de 10 souris non traitées.
Une augmentation de 3 secondes du temps de survie est à la fois significative statistiquement et reproductible.
Les $DE_{3''}$ des composés les plus actifs sont inférieures ou égales à 25 mg/kg par voie intrapéritonéale.

Les résultats des essais montrent que les composés de l'invention ont des propriétés neurotrophes et des propriétés neuroprotectrices.

Ils peuvent être utilisés pour le traitement des neuropathies périphériques, telles que les neuropathies traumatiques (section ou écrasement d'un nerfi ou ischémiques, les neuropathies métaboliques (diabète, urémie), les neuropathies infectieuses, alcooliques et médicamenteuses, les neuropathies génétiques, les affections du motoneurone telles que les amyotrophies spinales et la sclérose latérale amyotrophique.

Ils peuvent aussi être utilisés pour le traitement et la prévention de désordres cérébraux tels que ceux qui sont consécutifs, par exemple, à une attaque ischémique, un arrêt cardiaque ou respiratoire, une thrombose ou une embolie cérébrale, pour le traitement de la sénilité cérébrale, de la démence consécutive aux infarctus multiples, de la démence sénile, par exemple la maladie d'Alzheimer ou la maladie de Pick, pour le traitement de l'atrophie olivo-ponto-cérébellaire et d'autres maladies neurodégénératives telles que la chorée de Huntington, la sclérose latérale amyotrophique, pour le traitement des traumatismes crâniens ou spinaux, pour la prévention des dommages neuronaux faisant suite à des états convulsifs ou à la présence de tumeurs dans le système nerveux, pour le traitement des altérations neurologiques dues au SIDA, pour la prévention et le traitement des rétinopathies diabétiques, de la dégénérescence du nerf optique et des rétinopathies associées au glaucome.

Les composés de l'invention peuvent aussi être associés à des agents thrombolytiques tels que le rt-PA (activateur tissulaire du plasminogène recombiné, d'origine humaine) pour le traitement des infarctus cérébraux de type thromboenbolique, ou en association avec un composé diminuant la pression intraoculaire pour le traitement du glaucome, ou encore en association avec un agent anticancéreux, en vue de réduire les effets secondaires (neuropathies, etc) de ce dernier.

A cet effet ils peuvent être présentés sous toutes formes pharmaceutiques adaptées à l'administration entérale ou parentérale, en association avec des excipients appropriés, par exemple sous forme de comprimés, dragées, gélules, capsules, suppositoires, solutions ou suspensions buvables ou injectables, dosés pour permettre une administration journalière de 1 à 1000 mg de substance active.

## Revendications

1. Composé, éventuellement sous forme d'isomère optique pur ou d'un mélange de tels isomères, répondant à la formule générale (I)

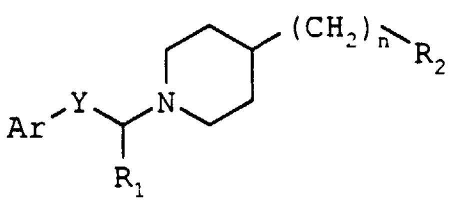

(I)

dans laquelle

Ar représente

a) un groupe phényle substitué par un atome d'halogène ou bien
b) un groupe de formule générale (I')

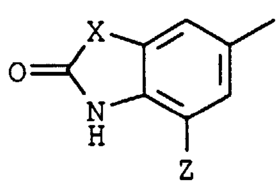

(I')

dans laquelle X représente un groupe de formule $-CH_2-$, $-(CH_2)_2-$ ou $-(CH_2)_3-$ et Z représente un atome d'hydrogène ou d'halogène ou un groupe méthyle,

Y représente un groupe de formule $-CH_2-$, -CO- ou -CHOH-,
$R_1$ représente un atome d'hydrogène ou un groupe méthyle,
$R_2$ représente un groupe cycloalkyle en $C_3$-$C_7$, et
n représente le nombre 0, 1, 2 ou 3,

à l'état de base libre ou de sel d'addition.

2. Procédé de préparation de composés selon la revendication 1, caractérisé en ce qu'on fait d'abord réagir une cétone halogénée de formule générale (II)

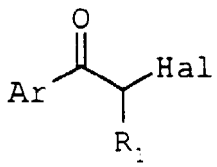

(II)

dans laquelle Ar est tel que défini dans la revendication 1 et Hal représente un atome d'halogène, avec une pipéridine substituée de formule générale (III)

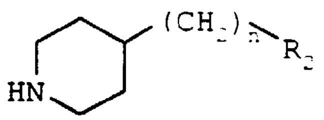

(III)

dans laquelle n et $R_2$ sont tels que définis dans la revendication 1, pour obtenir la cétone de formule générale (Ia)

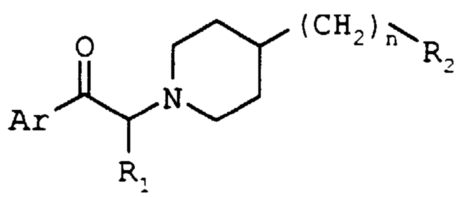

(Ia)

laquelle correspond à la formule générale (I) lorsque Y représente un groupe -CO-, puis si on le désire on réduit cette cétone pour obtenir l'alcool de formule générale (Ib)

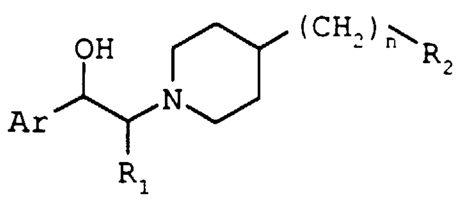

(Ib)

laquelle correspond à la formule générale (I) lorsque Y représente un groupe -CHOH-, puis si on désire obtenir un composé de formule générale (I) dans laquelle Y représente un groupe de formule $-CH_2-$ et Ar représente un groupe phényle substitué par un atome d'halogène on réduit ensuite l'alcool de formule générale (Ib), dans laquelle Y représente un groupe -CHOH- et Ar représente un groupe phényle substitué par un atome d'halogène, par réaction avec le chlorure de thionyle, suivie du traitement de l'intermédiaire chloré par l'hydrure de lithium et d'aluminium, ou bien, pour obtenir un composé de formule générale (I) dans laquelle Y représente un groupe de formule $-CH_2-$ et Ar représente un groupe de formule générale (I') telle que définie dans la revendication 1, on réduit d'abord une cétone halogénée de formule générale (II), dans laquelle Ar représente un groupe de formule générale (I') et Hal représente un atome d'halogène, au moyen de triéthylsilane et d'acide trifluoroacétique, pour obtenir le dérivé halogéné de formule générale (IV)

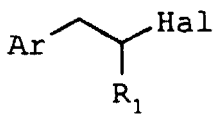

(IV)

puis on fait réagir ce dernier avec une pipéridine substituée de formule générale (III), dans laquelle n et $R_2$ sont tels que définis dans la revendication 1.

3. Médicament caractérisé en ce qu'il consiste en un composé selon la revendication 1.

4. Composition pharmaceutique caractérisée en ce qu'elle contient un composé selon la revendication 1, associé à un excipient.

**Patentansprüche**

1. Verbindung, gegebenenfalls in Form des reinen optischen Isomeren oder einer Mischung solcher Isomeren, der allgemeinen Formel (I)

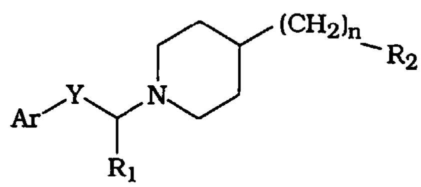

(I)

in der

Ar

a) eine durch ein Halogenatom substituierte Phenylgruppe oder
b) eine Gruppe der allgemeinen Formel (I')

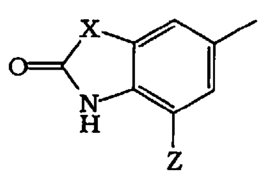

(I')

in der X eine Gruppe der Formel $-CH_2-$, $-(CH_2)_2-$ oder $-(CH_2)_3-$ und Z ein Wasserstoffatom, ein Halogenatom oder eine Methylgruppe darstellen,

Y eine Gruppe der Formeln $-CH_2-$, -CO- oder -CHOH-,
$R_1$ ein Wasserstoffatom oder eine Methylgruppe,
$R_2$ eine $C_3$-$C_7$-Cycloalkylgruppe und
n die Zahl 0, 1, 2 oder 3 bedeuten,

in Form der freien Base oder eines Additionssalzes.

2. Verfahren zur Herstellung der Verbindungen nach Anspruch 1, dadurch gekennzeichnet, daß man zunächst ein Halogenketon der allgemeinen Formel (II)

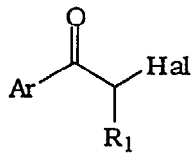

(II)

in der Ar die in Anspruch 1 angegebenen Bedeutungen besitzt und Hal für ein Halogenatom steht, mit einem substituierten Piperidin der allgemeinen Formel (III)

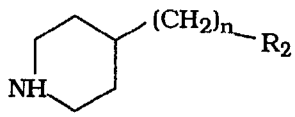

(III)

in der n und $R_2$ die in Anspruch 1 angegebebenen Bedeutungen besitzen, umsetzt zur Bildung des Ketons der allgemeinen Formel (Ia)

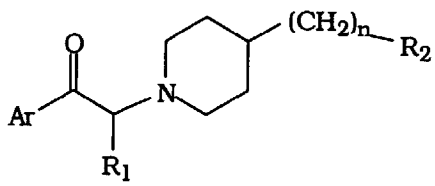

(Ia)

welche der allgemeinen Formel (I) entspricht, wenn Y eine Gruppe -CO- darstellt, und man dann gewünschtenfalls dieses Keton reduziert zur Bildung des Alkohols der allgemeinen Formel (Ib)

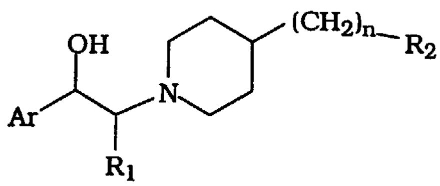

(Ib)

welche der allgemeinen Formel (I) entspricht, wenn Y eine Gruppe -CHOH- darstellt, und man dann, wenn man eine Verbindung der allgemeinen Formel (I) herstellen möchte, in der Y eine Gruppe der Formel $-CH_2-$ und Ar eine mit einem Halogenatom substituierte Phenylgruppe bedeuten, man anschließend den Alkohol der allgemeinen Formel (Ib), in der Y eine Gruppe -CHOH- und Ar eine durch ein Halogenatom substituierte Phenylgruppe darstellen, durch Reaktion mit Thionylchlorid, gefolgt von einer Behandlung des Chlor-Zwischenprodukts mit Lithiumaluminiumhydrid reduziert, oder zur Bildung einer Verbindung der allgemeinen Formel (I), in der Y eine Gruppe der Formel $-CH_2-$ und Ar eine Gruppe der allgemeinen Formel (I'), wie sie in Anspruch 1 definiert worden ist, darstellen, man zunächst ein Halogenketon der allgemeinen Formel (II), in der Ar eine Gruppe der allgemeinen Formel (I') und Hal ein Halogenatom darstellen, mit Hilfe von Triethylsilan und Trifluoressigsäure reduziert zur Bildung des Halogenderivats der allgemeinen Formel (IV)

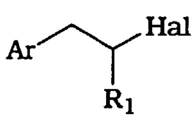

(IV)

welche Verbindung man dann mit einem substituierten Piperidin der allgemeinen Formel (III), in der n und $R_2$ die in Anspruch 1 angegebenen Bedeutungen besitzen, umsetzt.

3. Arzneimittel, dadurch gekennzeichnet, daß es aus einer Verbindung nach Anspruch 1 besteht.

4. Pharmazeutische Zubereitung, dadurch gekennzeichnet, daß sie eine Verbindung nach Anspruch 1 in Kombination mit einem Trägermaterial enthält.

**Claims**

1. Compound, optionally in the form of a pure optical isomer or a mixture of such isomers, corresponding to the general formula (I)

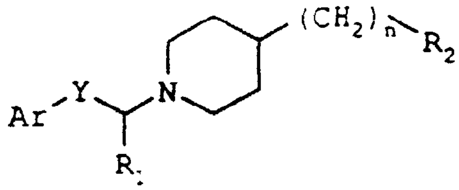

(I)

in which

Ar represents

a) a phenyl group substituted with a halogen atom or
b) a group of general formula (I')

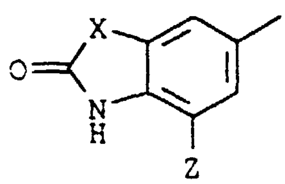

(I')

in which X represents a group of formula $-CH_2-$, $-(CH_2)_2-$ or $-(CH_2)_3-$ and Z represents a hydrogen or halogen atom or a methyl group,

Y represents a group of formula $-CH_2-$, -CO- or -CHOH-,
$R_1$ represents a hydrogen atom or a methyl group,
$R_2$ represents a $C_3$-$C_7$ cycloalkyl group, and
n represents the number 0, 1, 2 or 3,

in the form of the free base or an addition salt.

2. Process for the preparation of compounds according to Claim 1, characterized in that a haloketone of general formula (II)

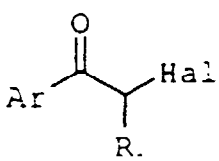

(II)

in which Ar is as defined in Claim 1 and Hal represents a halogen atom, is first reacted with a substituted piperidine of general formula (III)

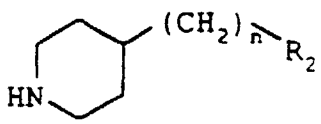

(III)

in which n and $R_2$ are as defined in Claim 1, to obtain the ketone of general formula (Ia)

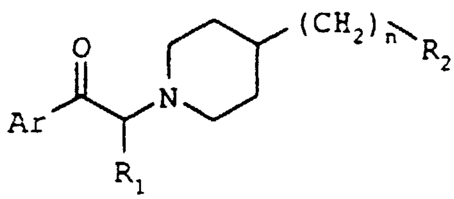

(Ia)

which corresponds to the general formula (I) when Y represents a -CO- group, followed, if so desired, by reduction of this ketone to obtain the alcohol of general formula (Ib)

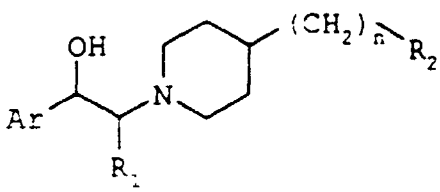

(Ib)

which corresponds to the general formula (I) when Y represents a -CHOH- group, then, if it is desired to obtain a compound of general formula (I) in which Y represents a group of formula $-CH_2-$ and Ar represents a phenyl group substituted with a halogen atom, the alcohol of general formula (Ib), in which Y represents a -CHOH- group and Ar represents a phenyl group substituted with a halogen atom, is reduced by reaction with thionyl chloride, followed by treatment of the chloro intermediate with lithium aluminium hydride, or alternatively, in order to obtain a compound of general formula (I) in which Y represents a group of formula $-CH_2-$ and Ar represents a group of general formula (I') as defined in Claim 1, a haloketone of general formula (II), in which Ar represents a group of general formula (I') and Hal represents a halogen atom, is first reduced using triethylsilane and trifluoroacetic acid, in order to obtain the halo derivative of general formula (IV)

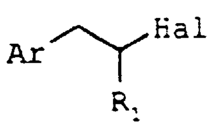

(IV)

followed by reaction of the latter with a substituted piperidine of general formula (III), in which n and $R_2$ are as defined in Claim 1.

3. Medicinal product, characterized in that it consists of a compound according to Claim 1.

4. Pharmaceutical composition, characterized in that it contains a compound according to Claim 1, combined with an excipient.